# EUROPÄISCHE PATENTSCHRIFT

(11) **EP 2 399 124 B1**
(45) Veröffentlichungstag und Bekanntmachung des Hinweises auf die Patenterteilung: **19.04.2017**
(21) Anmeldenummer: 10706582.3
(22) Anmeldetag: 18.02.2010
(51) Int. Cl.: G01N 33/00, G01N 27/66

(54) **VERFAHREN ZUM ERFASSEN DES KOHLENWASSERSTOFFANTEILS IN GASEN**
METHOD FOR DETECTING THE HYDROCARBON FRACTION IN GASES
PROCÉDÉ DE DÉTECTION DE LA TENEUR EN HYDROCARBURES DE GAZ

(30) Priorität: 18.02.2009 DE 102009009404
(43) Veröffentlichungstag der Anmeldung: 28.12.2011
(73) Patentinhaber: Beko Technologies GmbH, 41468 Neuss (DE)
(72) Erfinder: PENTH, Max, 66822 Lebach (DE); PENTH, Bernd, 66822 Lebach (DE); PARUSEL, Franz Josef, 40764 Langenfeld (DE)
(74) Vertreter: Patentanwälte Bauer Vorberg Kayser
(86) Internationale Anmeldenummer: PCT/EP2010/052080
(87) Internationale Veröffentlichungsnummer: WO 2010/094750

(56) Entgegenhaltungen:
- EP-A2- 0 488 120
- WO-A2-2010/075858
- US-A- 3 558 283
- US-A- 3 692 492
- US-A- 3 762 878
- US-A1- 2002 104 368
- PAPAMICHAIL N ET AL: "Monitoring of oil aerosol contamination in pressurised air with SnO2-based thick film sensors in real life conditions" SENSORS AND ACTUATORS B, ELSEVIER SEQUOIA S.A., LAUSANNE, CH LNKD- DOI:10.1016/J.SNB.2004.05.057, Bd. 106, Nr. 1, 29. April 2005 (2005-04-29), Seiten 61-66, XP025328513 ISSN: 0925-4005 [gefunden am 2005-04-29]

## Beschreibung

Die vorliegende Erfindung betrifft ein Verfahren zur Erfassung des Kohlenwasserstoffanteils in Gasen mit einem Messgerät. Derartige Messgeräte sind mit verschiedenen Sensortechnologien bekannt und dienen der Erfassung des Gehaltes von Öl, Kohlenwasserstoffen und oxidierbaren Gasen beispielsweise in Luft oder Druckluft. Häufig werden beispielsweise elektrisch beheizbare Halbleiter-Oxidmaterialien verwendet, die im beheizten Zustand ihren elektrischen Widerstand in Abhängigkeit von der Menge der in der Luft enthaltenen Kohlenwasserstoffe verändern.

Eine weitere Methode ist die Erfassung von Kohlenwasserstoffen mittels Elestoren. Dazu wird der zu messende Gasstrom über einen Körper aus beheiztem Katalysatormaterial geleitet, in dessen Inneren sich eine beheizte Platinwendel befindet. Die Kohlenwasserstoffkonzentration ist über die Änderung des elektrischen Widerstandes der beheizten und einer zweiten Platinwendel erfassbar, die sich durch die Verbrennungswärme des Kohlenwasserstoffanteils am Katalysator einstellt.

Ebenfalls bekannt ist die Verwendung von Flammenionisationsdetektoren. Bei derartigen Vorrichtungen werden die Kohlenwasserstoffen in einem Gasstrom verbrannt und die Spannungsänderung zwischen zwei Elektroden in der Flamme gemessen.

Eine weitere Methode ist die Erfassung der Kohlenwasserstoffkonzentration mittels Photoionisation. Dabei werden die Kohlenwasserstoffe mit ultraviolettem Licht bestrahlt. Die Energiemenge des Lichts muss dabei so hoch sein, dass Elektronen aus dem Kohlenwasserstoff herausgetrieben werden. Deren Anzahl lässt sich mit Elektroden messen.

Die oben genannten Verfahren eignen sich insbesondere für die Detektion höherer Konzentrationen in oxidierbaren Gasen, die Detektion geringerer Konzentrationen im unteren µg/Nm³-Bereich bzw. im ppb-Bereich ist aber nicht zuverlässig möglich.

Die mittels Photoionisationsdetektoren generierten Messwerte lassen nur indirekt auf die gemessene Stoffmenge schließen, da die Messwerte auch vom atomaren Aufbau der Verbindung abhängig sind und selbst bei gleichen Summenformeln recht stark variieren. Sofern die zu messende Verbindung aber konstant, bekannt und möglichst auch einheitlich ist, lässt sich die Konzentration des Kohlenwasserstoffanteils relativ zuverlässig messen. Allerdings sinkt die Messgenauigkeit mit abnehmender Konzentration an Kohlenwasserstoffen. Insbesondere steigt dabei der Einfluss des Feuchtegehalts der Luft. Mit abnehmendem Kohlenwasserstoffanteil wird der Einfluss der Luftfeuchte zunehmend größer, Messungen von Kohlenwasserstoffanteilen im unteren mg/Nm³-Bereich und insbesondere im µg/Nm³-Bereich sind nicht ausreichend genau durchzuführen.

Für die unterschiedlichen Anwendungen von Druckluft werden unterschiedliche Grenzwerte für den Ölanteil gefordert. Ölanteile bestehen aus tröpfchenförmigen Ölerosolen und aus Öldämpfen. Ölerosole und Öldämpfe können durch verschiedene Verfahren aus dem Druckluftstrom teilweise oder weitgehend eliminiert werden. Eine zeitnahe Messung von Öl in Druckluft ist aber bislang ein ungelöstes Problem.

Die Aufgabe der Erfindung besteht darin, ein gegenüber dem Stand der Technik verbessertes Verfahren zur Erfassung des Gehaltes von Öl, Kohlenwasserstoffanteilen und oxidierbaren Gasen in Gasen bereit zu stellen.

Die Aufgabe wird gelöst, durch ein Verfahren nach Anspruch 1. Vorteilhafte Ausführungsvarianten sich in den Unteransprüchen 2 bis 4 ausgeführt. In dem Verfahren führt ein dafür verwendetes Messgerät den zu messenden Gasstrom alternierend, beispielsweise mittels Magnetventilen, direkt oder über einen Referenzgaserzeuger zum Sensor. Als Referenzgaserzeuger ist beispielsweise ein Katalysator bzw. Oxidationskatalysator einsetzbar. Der Messwert wird auf diese Weise als Signaldifferenz zwischen dem Messgas und dem erzeugten Referenzgas, also dem oxidierten Messgas ermittelt.

In einer besonders vorteilhaften Ausführungsvariante beruht die Erfassung der Kohlenwasserstoffanteile auf dem Prinzip der Photoionisation. Das verwendete Messgerät besteht zu diesem Zweck aus zwei Hauptbestandteilen, einer Sensoreinheit mit Probenahmesonde und einer Auswerteeinheit mit Auswerteelektronik und Bedienoberfläche (Display). Die Bedienoberfläche kann gleichzeitig als Eingabeeinheit ausgeführt sein, beispielsweise durch einen Touchscreen. Die Sensoreinheit ist dabei über ein Signalkabel oder kabellos mit der Auswerteeinheit verbunden. Die Probenahmesonde kann vorzugsweise von oben zentrisch in eine Steigleitung montiert sein, so dass sie mittig aus dem zu überwachenden Gasstrom Gas entnehmen kann. Die Sensoreinheit weist definierte Fließwiderstände auf, die für einen konstanten Druck und einen konstanten Volumenstrom der einzelnen Messgase sorgen und beispielsweise durch eine Drossel mit definierter Bohrung, bzw. aus einem Sintermetall gebildet sind. Diese sind besonders wartungsarm und einfach zu reinigen. Weiterhin ist eine Alarmfunktion vorgesehen, die dem Benutzer bei zu niedrigem oder zu hohem Druck der Gasströme visuell oder akustisch informiert.

Das Messprinzip des Photoionisationsdetektors (PID) basiert, wie oben bereits angedeutet, auf der Ionisation der Gasmoleküle durch UV-Strahlung und der Erfassung des dabei entstehenden Ionenstroms. Die Stärke des Ionenstroms ist der Konzentration der ionisierten Moleküle direkt proportional. Das elektrische Signal ist somit messbar, kann elektronisch verstärkt und als Konzentration der gemessenen Substanzen im Display angezeigt werden.

Es hat sich gezeigt, dass die auf Grund der Oxidation im Referenzgas minimale Erhöhung der Feuchtigkeit dann vernachlässigbar ist, wenn bei der Messung Photoionisationslampen mit 10,6 eV verwendet werden, da bei diesen der Einfluss der Luftfeuchte am geringsten ist. Dem Referenzgasstrom wird trockenes Prüfgas beigemischt, um dadurch ein Referenzgas zu erhalten, das in seinem Feuchtigkeitsgehalt dem Messgas entspricht. Damit ist es möglich, die sogenannte Drift-Nulllinie und deren Einfluss auf das Messergebnis zu reduzieren. Die Drift-Nulllinie eines PID-Sensors ist verglichen mit anderen Sensortypen für Kohlenwasserstoffanteile nur klein, allerdings fällt sie bei kleiner werdenden Konzentrationen verstärkt ins Gewicht. Dadurch, dass der Messwert als Signaldifferenz zwischen dem Messgas und dem Referenzgas ermittelt wird und beide Messwerte gleichermaßen driften, hat die Drift des Sensors damit einen deutlich kleineren Einfluss auf das Messergebnis, insbesondere dann, wenn ein Mittelwert über viele Messungen gebildet wird. Eine lineare Drift und gleiche Schaltzeiten vorausgesetzt, ist die Wahrscheinlichkeit, dass die Drift im Mittelwert eliminiert wird relativ groß. Weiterhin ist es möglich, die Veränderung der Signalstärke durch Alterung und Verschmutzung des Messgeräts zuverlässig auszugleichen. Es hat sich nämlich gezeigt, dass die Messempfindlichkeit des Sensors in Abhängigkeit der Zeit, also insbesondere in Abhängigkeit der Alterung des Sensors, stark schwankt, wobei sich auch durch die Verschmutzung der UV-Lampe die Signalstärke allmählich ändert. Die Verschmutzung des Lampenfensters kann beispielsweise dadurch bewirkt werden, dass sich Inhaltsstoffe des Luftstroms auf der Fensteroberfläche niederschlagen und diese verkoken. Das verfahren ermöglicht eine Kalibrierung der Messempfindlichkeit über die Lebensdauer des Messgeräts dadurch, dass neben dem unveränderten Messgas und dem Referenzgas weiterhin ein Kalibriergas und ein Nullgas regelmäßig zur Neufestlegung der vom Sensor ausgehenden Signalstärke verwendet werden. Nullgas (z.B. synthetische Luft) enthält keine Kohlenwasserstoffe und keine Feuchtigkeit, Kalibriergas (z.B. Isobuten) dagegen weist einen definierten Kohlenwasserstoffanteil auf, allerdings ebenfalls keine oder nur eine ausgesprochen geringe Feuchtigkeit. Die beiden Hilfsgase werden mittels der Magnetventile alternierend zum Sensor geführt. Ein im verwendeten Messgerät vorgesehener

Datenspeicher beinhaltet sogenannte Responsefaktoren wichtiger und bekannter Prüfgase und deren Molekulargewichte. Aus der Differenz der Messergebnisse ist das Messgerät in der Lage, mit Hilfe eines entsprechenden Prozessors einen Faktor als Beziehung zwischen Messergebnissen und dem Prüfgas im Gewicht pro Volumen zu errechnen. Dieser Faktor, der sich im Laufe der Zeit zwangsweise ändert, ist als Kalibrierfaktor speicherbar, bzw. wird vom Messgerät automatisch gespeichert und dann der eigentlichen Messung zu Grunde gelegt. Die Kalibriermessung kann in regelmäßigen Abständen automatisch erfolgen, sie kann aber auch jederzeit vom Anwender eingeleitet werden. Es hat sich weiterhin gezeigt, dass die Luftfeuchte einen negativen Effekt auf die Signalstärke haben kann. Dies ist insbesondere dann der Fall, wenn die Luftfeuchtigkeit stark schwankt. Wenn Luft zum Beispiel hinter einem Kältetrockner austritt, sind die Schwankungen der Luftfeuchtigkeit zwar deutlich geringer, aber bei den äußerst geringen zu messenden Kohlenwasserstoffgehalten können auch diese nicht vernachlässigt werden. Aus diesem Grunde ist es möglich, eine Kalibriermessung zwischen dem Nullgas und dem Kalibriergas derart durchzuführen, dass der Einfluss der aktuellen Luftfeuchtigkeit eliminiert wird. Zu diesem Zweck werden die zur Kalibrierung verwendeten Hilfsgase mit der gleichen Luftfeuchtigkeit versehen, wie sie auch im Messgas vorliegt. Es wird im Gegensatz zu bekannten vergleichbaren Verfahren nicht ausschließlich auf handelsübliches Nullgas zur Kalibrierung zurückgegriffen, sondern es wird Kalibriergas verwendet, das durch Zuführung an den Referenzgaserzeuger in ein Nullgas umgewandelt wird. In diesem Fall ist der Begriff Nullgas nicht wörtlich, also im dem Sinne, dass keine Feuchtigkeit im Gas vorhanden ist, zu verstehen, sondern es steht zur Kalibrierung ein Referenzgas zur Verfügung, dass bis auf den Kohlenwasserstoffanteil die gleichen Inhaltsstoffe und exakt die gleiche Luftfeuchtigkeit aufweist, wie das eigentlich zu messende Messgas. Anstelle von handelsüblichem Prüfgas kann als Kalibriergas auch eine Mischung verwendet werden, die aus 90 - 100 % Referenzgas und entsprechend 0 - 10 % eines handelsüblichen Prüfgases gebildet ist. Auf diese Weise entsteht ein verdünntes Kalibriergas, das einen Feuchtigkeitsgehalt aufweist, der etwa dem des zu messenden Messgases entspricht. Durch die Erzeugung eines Kalibriergases vor Ort, kann es durch Verändern von Parametern in seiner Zusammensetzung entsprechend kontrolliert an die gegebenen Bedingungen angepasst werden. Dabei ist auch entscheidend, dass die Mengenzugabe an eingespeistem handelsüblichem Prüfgas in das Referenzgas gering ist und der Kohlenwasserstoffgehalt in der gleichen Größenordnung wie der erwartete Messbereich für den Kohlenwasserstoffgehalt im Messgas liegt.

Für eine Messung des Restölgehaltes im Bereich unterhalb der Klasse 1 gemäß DIN ISO 8573-1 (also < 10 µg Öl/Nm³ Gas bzw. Luft) wird zum Erzeugen des Kalibriergases möglichst nur eine geringe Menge an Prüfgas in den Referenzgasstrom eingeleitet, so dass sich beispielsweise als Konzentration ein Gehalt von <10 µg Isobuten/Nm³ Gas bzw. Luft ergibt, was weniger als 3,99 ppm Isobuten entspricht.

Die Durchflussmenge der verschiedenen Gasströme kann mit entsprechenden, ggfs. genormten Drosseln, Ventilen oder Durchflussreduktoren beeinflusst werden. Diese sind vorzugsweise austauschbar und in einer besonders vorteilhaften Ausführungsvariante regelbar, um damit zum Einen die Durchflussmenge zum Sensor einstellen und zum Anderen die gewünschten Mischverhältnisse der zu mischenden Gasströme zuverlässig gewährleisten zu können.

Als Referenzgaserzeuger können übliche Oxidationskatalysatoren eingesetzt werden, denkbar sind aber auch andere Vorrichtungen oder Verfahren zur Bereitstellung von Gasen mit den gewünschten Eigenschaften. Als Oxidationskatalysator dient beispielsweise platinierte Quarzwolle, die problemlos in einen dafür vorgesehenen Behälter einführbar ist. Ein Referenzgaserzeuger kann in ein Messgerät integriert sein, wodurch vor Ort lediglich die verschiedenen Fluid bzw. Gaszuführungen angeschlossen werden müssen. Ein derartiges Messgerät weist somit sämtliche Anschlüsse für entsprechende Gasleitungen und auch den elektrischen Anschluss auf, sodass es vor Ort flexibel an beliebigen Orten installierbar ist. Die Aufteilung des Messgeräts in die Sensoreinheit mit Probenahmesonde und die Auswerteeinheit mit Bedienoberfläche (Display) erweitert die Möglichkeiten einer räumlich flexiblen Aufstellung vor Ort zusätzlich. Die Auswerteeinheit mit Bedienoberfläche baut klein und kann nahezu überall, vorteilhafterweise an einer gut zugänglichen Position installiert werden, während die etwas größere Sensoreinheit räumlich getrennt von der Auswerteeinheit an der Messgasentnahmestelle angeordnet sein kann. Das verwendete Messgerät weist einen Eingang für ein Messgas auf, das entsprechend der Vorschrift zur isokinetischen Probenahme nach DIN/ISO 8753 aus einem strömenden Gas entnommen, aber nicht durch einen Strömungswiderstand geführt wird. Dadurch ist es möglich, dass der Sensor selbst bei relativ großen Gasmengen keinen Turbulenzen ausgesetzt ist, was zu Verfälschungen des Messsignals führen könnte. Durch eine vergleichende Messung mit einem Messgasstrom der durch einen Fließwiderstand geführt wurde, ist überprüfbar, ob die Analysen übereinstimmen. Es ist also möglich, die isokinetische Messung im Normalbetrieb weitgehend durch eine Messung mit vermindertem Gasfluss zu ersetzen.

Das verwendete Messgeräts kann mit einem ölfrei verdichtenden Kompressor zur Herstellung von Druckluft oder Druckgas verwendet werden, denkbar ist aber auch die Verwendung mit einem ölgeschmierten Kompressor, wenn diesem ein entsprechender Katalysator nachgeschaltet ist. Für Wartungsarbeiten ist vorzugsweise ein Bypass vorgesehen.

Im Rahmen dieser Erfindung wird zwischen den folgenden Gasströmen unterschieden. Der Begriff Messgas beschreibt das zu messende Gas, beispielsweise Umgebungsluft. Als Nullgas wird ein Gasstrom bezeichnet, der keine Feuchtigkeit und keine Kohlenwasserstoffe aufweist. Referenzgas ist derjenige Gasstrom, der vom Messgas abgezweigt und über einen Referenzgaserzeuger, beispielsweise ein Oxidationskatalysator geleitet wurde. Kalibriergas bezeichnet ein Gas, das zur Kalibrierung des Messgerätes genutzt wird und eine definierte Menge von Kohlenwasserstoffen enthält. Prüfgas ist ein käuflich zu erwerbendes und definiertes Gas, es kann aber auch ein Mischgas beispielsweise aus Nullgas und einem kleinen Anteil sensoraktivem Gas sein.

Die Dauer der Zuführung der Gasströme zu dem Sensor kann an die Gegebenheiten vor Ort angepasst werden, eine alternierende Zuführung über einer Dauer von etwa 20 Sekunden hat sich aber als zielführend erwiesen.

Die Erfindung wird im Folgenden mit Bezug auf die beiliegenden Figuren näher erläutert. Es zeigen:
- Fig. 1:: Eine perspektivische Darstellung eines verwendeten nicht erfindungsgemäßen Messgerätes,
- Fig. 2:: ein Funktionsschema, bzw. Schaltbild der Fluidströme des Messgeräts,
- Fig. 3_{:}: eine verwendete nicht erfindungsgemäße Sensoreinheit ohne umgebendes Gehäuse.

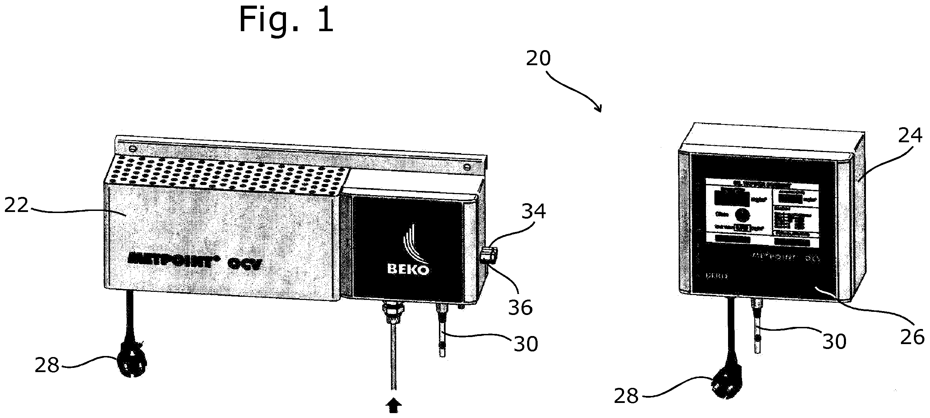

Figur 1 zeigt ein verwendtes nicht erfindungsgemäßes Messgerät 20, das aus einer Sensoreinheit 22 und einer Auswerteinheit 24 gebildet ist. Die Auswerteinheit 24 weist weiterhin eine Bedienoberfläche 26, ausgeführt als Touchscreen, auf. Beide Geräte sind mit einem Stecker 28 für einen elektrischen Anschluss ausgerüstet.

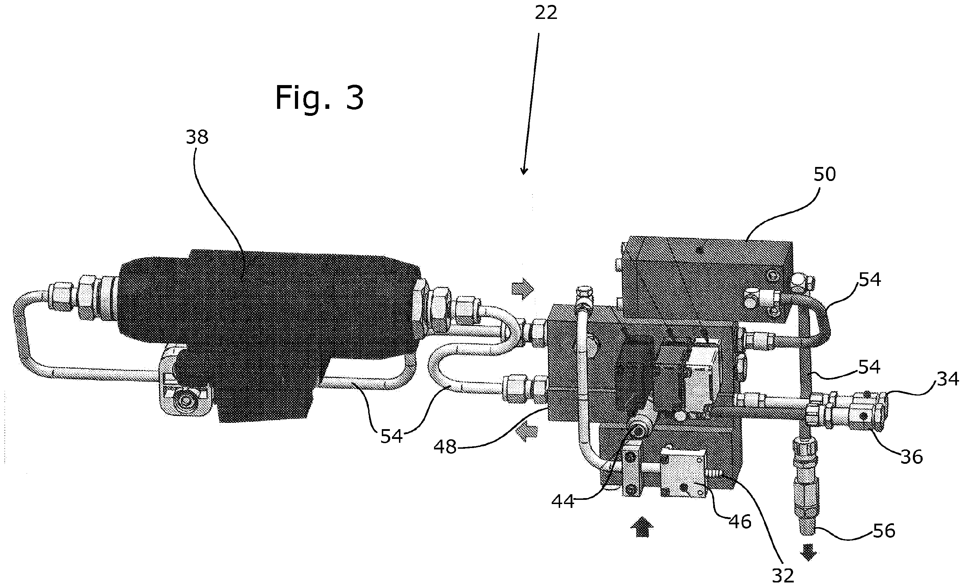

Weiterhin weisen beide Geräte Signalkabelanschlüsse 30 auf, über die sie miteinander verbindbar sind. Anstelle der Signalkabelanschlüsse 30 ist aber auch eine kabellose Funkverbindung denkbar und die Installation je nach Gegebenheiten vor Ort deutlich erleichtert. Weiterhin ist erkennbar, dass die Sensoreinheit 22 einen Messgasanschluss 32 aufweist, über den das zu messende Gas zugeführt wird. Weiterhin ist ein Nullgasanschluss 34 für die Zuführung von Nullgas und ein Kalibriergasanschluss 36 für eine Zuführung von Kalibriergas erkennbar. Die Bedienoberfläche 26 dient nicht nur der Bedienung des Messgeräts 20, im gezeigten Ausführungsbeispiel stellt sie auch die Anzeige bzw. das Display des Messgeräts 20 dar. Innerhalb der Sensoreinheit 22 ist eine nicht erkennbare Referenzgaseinheit 38 angeordnet (vergleiche Figur 3), die als Oxidationskatalysator ausgeführt sein kann.

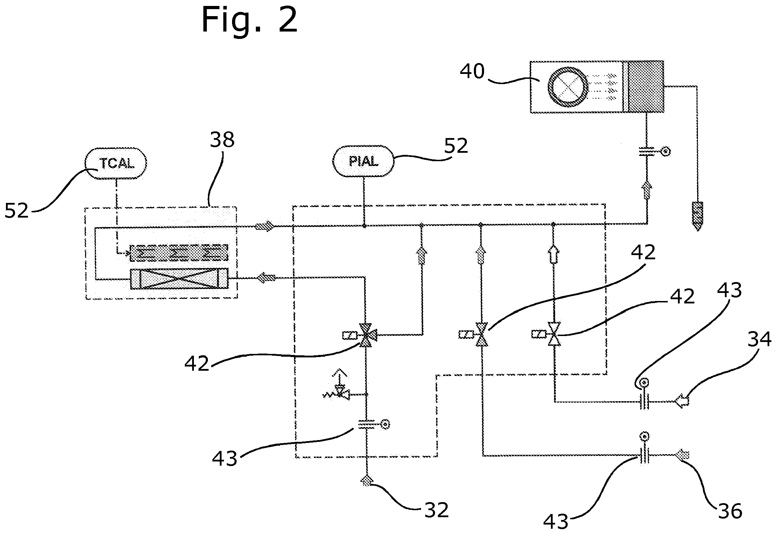

Aus Figur 2 wird die Funktion des Messgeräts deutlich. Erkennbar sind die drei Gaszuführungen, nämlich der Messgasanschluss 32, der Nullgasanschluss 34 und der Kalibriergasanschluss 36. Weiterhin sind die Referenzgaseinheit 38 und ein Sensor 40, ausgeführt als Photoionisationsdetektor, in einer Messkammer 50 erkennbar. Die verschiedenen Gasströme werden über Ventile 42 geschaltet, bzw. geregelt. Die Ventile 42 sind vorzugsweise als Magnetventile ausgeführt. Demnach ist es möglich, das zu untersuchende Messgas über den Messgasanschluss 32 zur Referenzgaseinheit und von dort als Referenzgas zum Sensor 40 zu leiten. Alternativ ist es aber auch möglich, das Messgas unmittelbar zum Sensor 40 zu leiten. Auch ist es möglich, Kalibriergas oder auch Nullgas gemeinsam oder im Wechsel dem Sensor 40 zuzuführen.

Fließwiderstände 43 gewährleisten einen konstanten Druck und einen konstanten Volumenstrom.

Schließlich sind Überwachungselemente 52 vorgesehen, die beispielsweise dann Alarm geben, wenn der Betriebsdruck im Messgerät 20 zu hoch wird.

Die Auswerteeinheit 24 kann geeignete Anschlüsse für einen Rechner oder eine Datenbank aufweisen, es können auch Anschlüsse für Datenspeicher, wie SD-Karten, USB-Sticks oder ähnliches vorhanden sein. Zur Datenspeicherung weist die Auswerteinheit 24 weiterhin einen Datenspeicher und zur Berechnung und Auswertung der Messdaten einen entsprechenden Prozessor auf.

Die Referenzgaseinheit 38 ist über Leitungen 54 mit dem Ventilgehäuse 48 und der Messkammer 50 verbunden. Eine weitere Leitung 54 führt aus der Messkammer 50 heraus und endet in einem Auslass 56. Weiterhin ist die Messkammer 50 mit dem Ventilgehäuse 48 verbunden.

Figur 3 verdeutlicht den kompakten Aufbau der Sensoreinheit 22 mit integrierter Referenzgaseinheit 38. Erkennbar sind insbesondere der Nullgasanschluss 34 und der Kalibriergasanschluss 36, die Referenzgaseinheit 38, der Sensor 40 sowie ein Sicherheitsventil 44. Ein Strömungswächter 46 mit entsprechendem Druckschalter ist ebenfalls vorgesehen. Innerhalb eines Ventilgehäuses 48 sind die Ventile 42 und weitere elektrische und elektronische Komponenten untergebracht. Der Sensor 40 ist innerhalb einer Messkammer 50 angeordnet. Die Fließwiderstände 43 (Drosseln) sind in den jeweiligen Anschlussverschraubungen der Gasanschlüsse 32, 34, 36 integriert und deswegen nicht erkennbar.

## Patentansprüche

1. Verfahren zum Erfassen des Kohlenwasserstoffanteils in einem Gasstrom, wobei einem Messgerät (20) Messgas über einen Messgasanschluss (32) einer Sensoreinheit (22) zugeführt und in einen ersten Gasstrom und einen zweiten Gasstrom aufgeteilt wird, indem der Gasstrom des Messgases mittels eines Ventils (42) alternierend als Messgasstrom direkt einem Sensor (40) oder über einen Oxidationskatalysator (38) als Referenzgasstrom dem Sensor (40) zugeführt wird, wobei die Sensoreinheit (22) dem Referenzgasstrom zwischen dem Oxidationskatalysator (38) und dem Sensor (40) über einen Nullgasanschluss (34) und ein weiteres Ventil (42) Nullgas als trockenes Prüfgas beimischt, wobei das Nullgas keine Kohlenwasserstoffe und keine Feuchtigkeit enthält, und mit ihm ein Referenzgas erhalten wird, das in seinem Feuchtigkeitsgehalt dem Messgas entspricht, und eine Auswerteeinheit (24) den Kohlenwasserstoffanteil im Gasstrom des Messgases über eine Signaldifferenz zwischen dem Messgas und dem Referenzgas ermittelt und auf der Anzeige der Auswerteeinheit (24) anzeigt.

2. Verfahren nach Anspruch 1, **dadurch gekennzeichnet, dass** der Sensor (40) als Photoionisationssensor ausgeführt ist.

3. Verfahren nach einem der Ansprüche 1 und 2, **dadurch gekennzeichnet, dass** Fließwiderstände (43) vorgesehen sind, die einen konstanten Druck und einen konstanten Volumenstrom der zugeführten Gase gewährleistet.

4. Verfahren nach einem der Ansprüche 1 bis 3, **dadurch gekennzeichnet, dass** weiterhin ein Kalibriergasanschluss (36) vorgesehen ist und Kalibrierungsvorgänge durchgeführt werden, indem ein Kalibriergas und ein Nullgas zur Neufestlegung der vom Sensor (40) ausgehenden Signalstärke verwendet werden, wobei das Nullgas keine Kohlenwasserstoffe und keine Feuchtigkeit enthält und das Kalibriergas einen definierten Kohlenwasserstoffanteil aufweist, und diese beiden Hilfsgase alternierend dem Sensor (40) zugeführt werden.

## Claims

1. A method for detecting the hydrocarbon content in a gas stream, wherein measuring gas is fed to a measuring device (20) via a measuring gas connector (32) of a sensor unit (22) and is divided into a first gas stream and a second gas stream by the gas stream of the measuring gas being fed, in an alternating manner by means of a valve (42), directly to the sensor (40) as a measuring gas stream, or to the sensor (40) as a reference gas stream via an oxidation catalyst (38), wherein the sensor unit (22) admixes zero gas as a dry test gas to the reference gas stream via a zero gas connector (34) and another valve (42) between the oxidation catalyst (38) and the sensor (40), wherein the zero gas contains no hydrocarbons and no humidity, and a reference gas is obtained with it whose humidity content corresponds to the measuring gas, and an evaluation unit (24) determines the hydrocarbon content in the gas stream of the measuring gas through a signal difference between the measuring gas and the reference gas and displays it on the display of the evaluation unit (24).

2. The method according to claim 1, **characterized in that** the sensor (40) is configured as a photoionization sensor.

3. The method according to any one of the claims 1 and 2, **characterized in that** flow resistors (43) are provided which ensure a constant pressure and a constant volumetric flow rate of the supplied gases.

4. The method according to any one of the claims 1 to 3, **characterized in that**, further, a calibration gas connector (36) is provided and calibration processes can be carried out by a calibration gas and a zero gas being used for re-determining the signal strength (40) provided by the sensor, wherein the zero gas contains no hydrocarbons and no humidity and the calibration gas has a defined hydrocarbon content, and these two auxiliary gases are alternately fed to the sensor (40).

## Revendications

1. Procédé destiné à détecter la proportion d'hydrocarbures dans un courant de gaz, dans lequel un gaz de mesure est amené à un appareil de mesure (20) via un raccord de gaz de mesure (32) d'une unité capteur (22) et est divisé en un premier courant de gaz et en un deuxième courant de gaz en amenant le courant de gaz du gaz de mesure au moyen d'une vanne (42) de manière alternée en tant que courant de gaz de mesure directement à un capteur (40) ou via un catalyseur d'oxydation (38) en tant que courant de gaz de référence au capteur (40), dans lequel ladite unité capteur (22) ajoute du gaz de zéro en tant que gaz sec d'essai au courant de gaz de référence, entre ledit catalyseur d'oxydation (38) et ledit capteur (40), via un raccord de gaz de zéro (34) et une autre vanne (42), ledit gaz de zéro ne contenant pas d'hydrocarbures et pas d'humidité, et on obtient avec celui-ci un gaz de référence qui, quant à sa teneur en humidité, correspond au gaz de mesure, et une unité d'évaluation (24) détermine la proportion d'hydrocarbures dans le courant de gaz du gaz de mesure par une différence de signal entre le gaz de mesure et le gaz de référence et affiche celle-ci sur le dispositif d'affichage de l'unité d'évaluation (24).

2. Procédé selon la revendication 1, **caractérisé par le fait que** le capteur (40) est réalisé en tant que capteur de photoionisation.

3. Procédé selon l'une quelconque des revendications 1 et 2, **caractérisé par le fait que** des résistances d'écoulement (43) sont prévues qui garantissent une pression constante et un débit volumique constant des gaz amenés.

4. Procédé selon l'une quelconque des revendications 1 à 3, **caractérisé par le fait que**, en outre, un raccord de gaz de calibrage (36) est prévu et des opérations de calibrage sont mises en oeuvre en utilisant un gaz de calibrage et un gaz de zéro pour la nouvelle fixation de l'intensité de signal émanant du capteur (40), le gaz de zéro ne contenant pas d'hydrocarbures et pas d'humidité, et le gaz de calibrage présentant une proportion définie d'hydrocarbures, et ces deux gaz auxiliaires étant amenés de manière alternée au capteur (40).
